# EUROPEAN PATENT SPECIFICATION

(11) **EP 4 285 817 B1**
(45) Date of publication and mention of the grant of the patent: **06.05.2026**
(21) Application number: 21923023.2
(22) Date of filing: 29.09.2021
(51) Int. Cl.: A61B 5/08, A61B 5/113, A61B 5/00

(54) **SLEEP MONITORING CAPSULE AND SLEEP MONITORING SYSTEM**
SCHLAFÜBERWACHUNGSKAPSEL UND SCHLAFÜBERWACHUNGSSYSTEM
CAPSULE DE SURVEILLANCE DU SOMMEIL ET SYSTÈME DE SURVEILLANCE DU SOMMEIL

(30) Priority: 29.01.2021 JP 2021013416
(43) Date of publication of application: 06.12.2023
(73) Proprietor: Nine Hours, Inc., Tokyo 101-0054 (JP)
(72) Inventor: MATSUI Takahiro, Tokyo 101-0054 (JP); ARITA Hideyuki, Tokyo 101-0054 (JP); NOZUE Kaoru, Tokyo 101-0054 (JP)
(74) Representative: Papula Oy
(86) International application number: PCT/JP2021/035841
(87) International publication number: WO 2022/163016

(56) References cited:
- AU-A4- 2019 101 529
- CN-A- 104 720 766
- CN-A- 109 668 230
- JP-A- 2002 153 432
- JP-A- 2003 322 383
- JP-A- 2004 344 265
- JP-A- H06 237 902

## Description

### TECHNICAL FIELD

The present invention relates to a sleep monitoring capsule and a sleep monitoring system including a capsule for defining a sleep space for one person and monitoring a sleep state of a user sleeping in the capsule.

### BACKGROUND ART

In recent years, attention has been paid to preventive medicines that find and resolve onset of disease before a disease occurs or before a disease deteriorates, rather than treatment after a disease.

It Is also known that diseases such as depression and schizophrenia are caused by a decrease in quality of sleep, and diseases such as arteriosclerosis, myocardial infarction, and stroke are caused by apnea syndrome during sleep. Therefore, a sleep sensor for monitoring a state of a person during sleep has been proposed.

For example, Patent Document 1 discloses a sleep sensor that is attached to a subject and configured to obtain measurement data relating to pulse waves or pulse waves and oxygen saturation in the blood of a subject, obtain measurement data relating to body temperature or body surface temperature of the subject, obtain measurement data relating to body motion of the subject, and obtain measurement data relating to sounds or voices emitted by the subject or sounds in the surrounding environment.

Patent Document 1: Japanese Unexamined Patent Application, Publication No. 2013-150660

Document CN 104 720 766 discloses a sleep monitoring capsule having a capsule that demarcates a sleep space for one person for monitoring a sleep state of a user sleeping in the capsule, the sleep monitoring capsule comprising a monitoring unit that monitors a state of the user sleeping in the capsule.

### DISCLOSURE OF THE INVENTION

### Problems to be Solved by the Invention

However, when a sleep sensor is attached to a subject as in Patent Document 1, there is a problem in that the measurement data measured by measuring the state of the user during sleep varies depending on the environment where the subject is sleeping, and the reliability of the measurement data decreases.

The present invention has been made in view of the abovementioned situations, and it is an object of the present invention to provide a sleep monitoring capsule and a sleep monitoring system capable of improving the reliability of measurement data obtained by measuring a state of a user during sleep.

### Unit for Solving the Problems

(1) A sleep monitoring according to claim 1 having a capsule that demarcates a sleep space for one person for monitoring a sleep state of a user sleeping in the capsule, the sleep monitoring capsule comprising a monitoring unit that monitors a state of the user sleeping in the capsule.

In the invention according to (1), the sleep monitoring capsule includes a capsule which demarcates a sleep space for one person, and a monitoring unit which monitors a sleep state of a user who sleeps in the capsule.

According to the invention of (1), the state of the user during sleep is monitored in the capsule demarcating the sleep space for one person.

Thus, by demarcating the sleep space by the capsule in advance, it is possible to suppress the influence of the environment outside the capsule on the state of the user during sleep and the measurement data obtained by measuring the state of the user. Therefore, it is possible to provide a sleep monitoring capsule capable of improving the reliability of measurement data obtained by measuring the state of the user during sleep.

(2) The sleep monitoring capsule according to (1), wherein the capsule includes a door that is capable of opening and closing an entrance/exit from an inner side of the capsule, and a ventilation unit that ventilates inside of the capsule.

In the invention (2), the capsule includes a door (for example, a lid, a shutter, or the like) and a ventilation unit. The door can open and close the entrance and exit of the capsule from the interior side of the capsule. The ventilation unit ventilates the capsule.

According to the invention of (2), the entrance/exit of the capsule is closed by the door from the inner side of the capsule, whereby it is possible to improve the sound insulating property of the inside and outside of the capsule. Thus, it is possible to suppress noise mixed in the measurement data obtained by measuring the state of the user during sleep. In addition, since the ventilation unit is provided, even when the airtightness of the door is improved in order to improve the sound insulating properties of the inside and outside of the capsule, it is still possible to keep the environment such as the oxygen concentration and the temperature in the capsule constant by ventilation by the ventilation unit. Therefore, it is possible to provide a sleep monitoring capsule capable of improving the reliability of measurement data obtained by measuring the state of the user during sleep.

(3) A sleep monitoring system comprising: a sleep monitoring capsule according to (1) or (2); and a controller that determines a state of a user during sleep, wherein the controller includes an acquisition unit that acquires sleep information indicating the state of the user during sleep from the monitoring unit, and an analysis unit that analyzes the state of the user during sleep based on the sleep information.

The invention of (3) includes a sleep monitoring capsule and a controller. The controller determines a state of the user during sleep and includes an acquisition unit and an analysis unit. The acquisition unit acquires sleep information indicating the state of the user during sleep from the monitoring unit. The analysis unit analyzes the state of the user during sleep based on the sleep information.

According to the invention of (3), the state of the user during sleep can be analyzed based on the sleep information acquired from the monitoring unit of the sleep monitoring capsule according to (1) or (2). Thus, since the sleep space is demarcated in advance by the capsule, and the state of the user during sleep is analyzed based on the sleep information of the user during sleep in the closed sleep space, it is possible to improve the accuracy of the analysis.

(4) The sleep monitoring system according to (3), wherein the controller further includes a classification unit that classifies the state of the user during sleep analyzed by the analysis unit for each disease that is closely related to sleep.

Here, diseases that are closely related to sleep include high blood pressure, sleep apnea syndrome, REM sleep behavior disorder, PLMD (periodic limb movement disorder), frequent urine, predisposition toward allergies, and the like, and also include myocardial and cerebral infarction, Parkinson's disease, depression insomnia, and the like induced from these diseases. The result of analyzing the state of the user during sleep is useful for diagnosing these diseases. The state of the user during sleep, which is useful information for diagnosing these diseases, varies for each disease. There are also different professionals who diagnose these diseases.

According to the invention of (4), by classifying the state of the user during sleep, which is the analysis result, for every disease that is closely related to sleep, it is possible to efficiently use the analysis result, and it is also possible to improve the accuracy of diagnosis of the professional practitioner in the future.

(5) The sleep monitoring system according to (3) or (4), wherein the monitoring unit includes a plurality of detection units that each detect the state of the user during sleep, and the analysis unit analyzes the state of the user during sleep by mutually complementing a plurality of pieces of sleep information detected by the plurality of detection units.

Here, in a case of analyzing the biometric information as the state of the user during sleep, how to set the basis of the biometric information is important. If the analysis is performed based on the sleep information detected by one detection unit, the analysis result varies depending on the setting of the basis.

According to the invention of (5), since the plurality of pieces of sleep information respectively detected by the plurality of detection unit are mutually complementing and the state of the user during sleep is analyzed, analysis can be performed based on mutual numerical changes in the plurality of pieces of sleep information, and thus, it is possible to improve the accuracy of analysis.

(6) The sleep monitoring system according to any one of (3) to (5), wherein the monitoring unit acquires body information relating to a body of the user, the acquisition unit acquires the body information from the monitoring unit, and the analysis unit performs correction based on the body information to analyze the state of the user during sleep.

Here, in a case of analyzing the biometric information as the state of the user during sleep, the biometric information varies widely from person to person, and it may be difficult to accurately analyze the biometric information in accordance with the user by comparing the biometric information with a uniform reference value.

According to the invention of (6), since the correction based on the body information relating to the body of the user is performed and the state of the user during sleep is analyzed, analysis according to the user becomes possible, and the accuracy of analysis is improved.

### Effects of the Invention

According to the present invention, it is possible to provide a sleep monitoring capsule and a sleep monitoring system capable of improving reliability of measurement data obtained by measuring a state of a user during sleep.

### BRIEF DESCRIPTION OF THE DRAWINGS

FIG. 1 is a diagram illustrating an outline of a sleep monitoring capsule according to an embodiment of the present invention;
FIG. 2 is a diagram illustrating an outline of a sleep monitoring system according to the embodiment of the present invention;
FIG. 3 is a diagram showing a functional configuration of the sleep monitoring system according to the embodiment of the present invention;
FIG. 4 is a diagram illustrating analysis by an analysis unit according to the embodiment of the present invention;
FIG. 5 is a diagram illustrating analysis by analysis unit according to the embodiment of the present invention;
FIG. 6 is a diagram illustrating analysis by an analysis unit according to the embodiment of the present invention;
FIG. 7 is a diagram illustrating analysis by an analysis unit according to the embodiment of the present invention;
FIG. 8 is a table illustrating an outline of classification by a classification unit according to the embodiment of the present invention;
FIG. 9 is a diagram illustrating an outline of a sleep monitoring capsule according to an application example of the embodiment of the present invention;
FIG. 10 is a diagram illustrating an outline of a sleep monitoring system according to the application example of the embodiment of the present invention;
FIG. 11 is a diagram showing a functional configuration of a sleep monitoring system according to the application example of the embodiment of the present invention; and
FIG. 12 is a diagram illustrating functions of an analysis unit and a classification unit according to the application example of the embodiment of the present invention.

### PREFERRED MODE FOR CARRYING OUT THE INVENTION

### Present Embodiment

An embodiment (hereinafter, referred to as present embodiment) of the present invention will now be described in detail with reference to the accompanying drawings. In the following drawings, like elements are denoted by like reference numerals throughout the description of embodiments.

### Basic Configuration of Sleep Monitoring Capsule

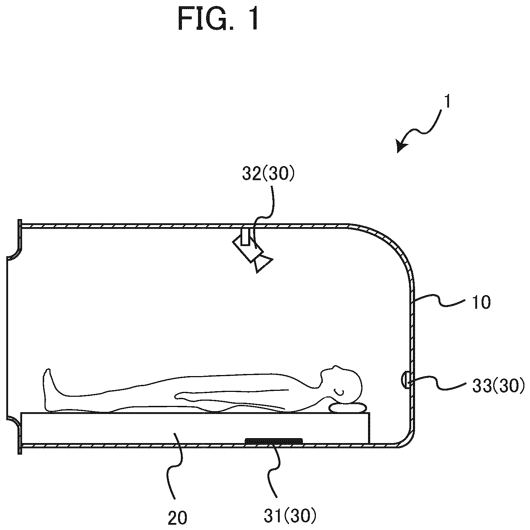

FIG. 1 is a diagram illustrating an outline of a sleep monitoring capsule according to an embodiment of the present invention. The sleep monitoring capsule 1 includes a capsule 10 for demarcating a sleep space (a bedroom) for one person, a sleep tool 20 placed in the capsule 10 for a user lying down thereon, and a monitoring unit 30 in the capsule 10 for monitoring a state of the user during sleep on the sleep tool 20.

A plurality of sleep monitoring capsules 1 are installed in an accommodation facility such as a so-called capsule hotel. However, the present invention is not limited thereto, and a plurality of sleep monitoring capsules 1 may be installed in a hospital, a residence, a school, a store, etc.

The capsule 10 is made of, for example, a material such as FRP (Fiber Reinforced Plastics), plastic, carbon, steel panel, or wood, is also called a capsule bed, is formed in a cylindrical shape forming a sleep space inside, and has an opening provided at one end face for the user to enter inside. The capsule 10 is not limited to a cylindrical shape as long as it can demarcate a single sleep space, and may be formed into a box shape, or may have any size.

The sleep tool 20 is, for example, a sleep tool such as a bed, mattress, or futon, and is provided such that the longitudinal direction thereof is along the longitudinal side of the capsule 10. For the sleep tool 20, it is preferable to provide a pillow in the vicinity of an end portion in a predetermined direction. This makes it possible to define the orientation in which the user lies down with respect to the capsule 10 and the direction in which the user lies down with respect to the capsule 10.

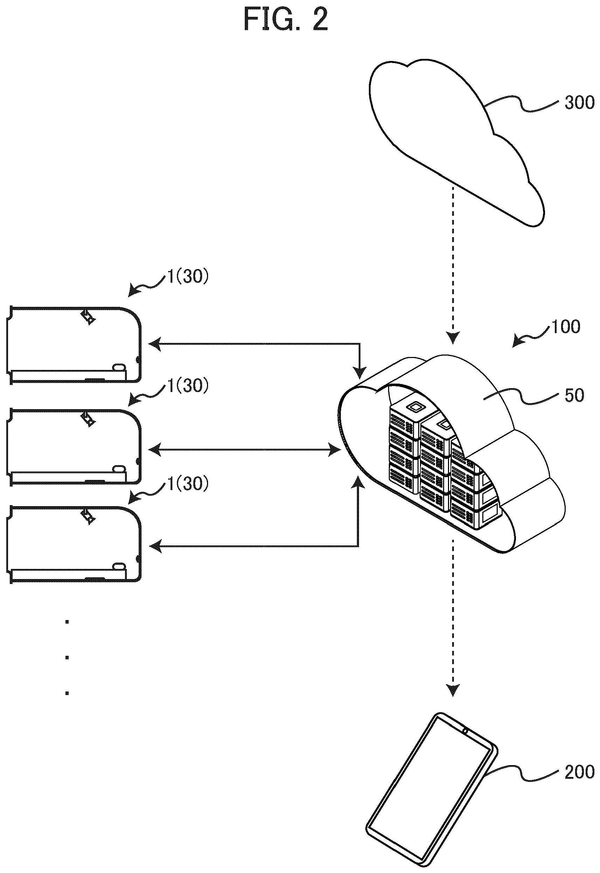

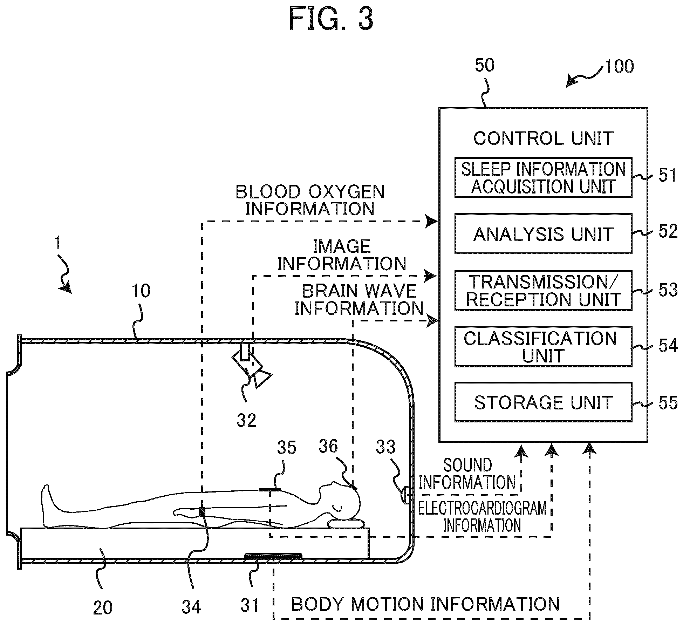

Although details will be described later, the monitoring unit 30 includes, for example, a body motion detection unit 31, an imaging unit 32, a sound collecting unit 33, and a monitoring control unit that acquires sleep information indicating a state of a user during sleep by these units and transmits the information to a control unit 50 (see FIGS. 2 and 3) described later. Although not shown, the monitoring control unit may be provided in each sleep monitoring capsule 1, or may be a device (computer) installed in an accommodation facility (e.g., capsule hotel) in which the sleep monitoring capsules 1 are installed and connected to each unit.

The body motion detection unit 31 includes, for example, a pressure sensor, an acceleration sensor, or the like, and is provided so as to be in contact with a predetermined position of the sleep tool 20 (e.g., a position where the back of the user comes into contact when the user is lying down) (e.g., on a lower surface or an upper surface of the sleep tool 20), and detects movement of the body of the user transmitted to the sleep tool 20.

The imaging unit 32 includes, for example, a camera, is fixed to an inner wall of the capsule 10 (for example, an inner wall including a ceiling, or the like), images at least the head of the user lying down on the sleeping tool 20, and generates moving image data and still image data.

The sound collecting unit 33 includes, for example, a microphone, is fixed to an inner wall of the capsule 10 (for example, an inner wall including an end face on the head side of the user), and collects sound in the capsule 10 during sleep by the user with the sleep tool 20.

With such a configuration, the sleep monitoring capsule 1 demarcates the sleep space by the capsule 10 in advance, whereby it is possible to suppress the influence of the environment outside the capsule 10 on the state of the user during sleep and the measurement data obtained by measuring the state of the user. Furthermore, in the capsule 10, by defining the position where the user lies down by the sleep tool 20, it is possible to suppress the influence of the position where the user is lying down on the state of the user during sleep and the measurement data obtained by measuring the state of the user. Therefore, it is possible to improve the reliability of the measurement data obtained by measuring the state of the user during sleep.

### Overall Configuration of Sleep Monitoring System

FIG. 2 is a diagram illustrating an outline of a sleep monitoring system according to an embodiment of the present invention. The sleep monitoring system 100 includes a sleep monitoring capsule 1 and a control unit 50 connected to the sleep monitoring capsule 1 via a network.

In the present embodiment, the control unit 50 is configured by, for example, a cloud server connected to the monitoring unit 30 of the sleep monitoring capsule 1 via the Internet; however, the present invention is not limited thereto, and may be, for example, an apparatus (computer) installed in an accommodation facility (for example, capsule hotel or the like) in which the sleep monitoring capsule 1 is installed. In this case, the apparatus may function as a monitoring control unit of the monitoring unit 30.

A plurality of sleep monitoring capsules 1 can be connected to the control unit 50. Furthermore, the control unit 50 can be connected to a terminal 200 outside the system and a server 300 outside the system via a network.

The terminal 200 is, for example, a smartphone, a tablet, or a personal computer, and is a terminal operated by a user of the sleep monitoring capsule 1. The user can receive the sleep information in the sleep monitoring capsule 1, the determination result based on the sleep information, and the like at the terminal 200, and confirm the information.

The server 300 is, for example, a server of an accommodation facility in which the sleep monitoring capsules 1 are installed. Examples of the server 300 include a server for storing customer data of the accommodation facility and a server of a medical institution in which medical diagnosis of sleep information can be performed such as a medical institution and a university, or the like.

### Functional Configuration of Sleep Monitoring System

FIG. 3 is a diagram showing a functional configuration of the sleep monitoring system according to the embodiment of the present invention. In the sleep monitoring system 100, the sleep monitoring capsule 1 includes, as the monitoring unit 30, a blood oxygen measuring unit 34, an electrocardiogram measuring unit 35, and a brain wave measuring unit 36, in addition to the body motion detection unit 31, the imaging unit 32, and the sound collecting unit 33.

The blood oxygen measuring unit 34 is, for example, a wristwatch-type sensor, and measures blood oxygen of the user when the user is worn on the wrist.

The electrocardiogram measuring unit 35 is, for example, an electrocardiogram sensor, is provided on the chest of the user, and measures electrical activity of the user's heart.

The brain wave measuring unit 36 is, for example, a brain wave sensor, and is provided on the head of the user to measure electrical activity of the brain of the user.

Furthermore, the imaging unit 32 includes an infrared camera. With such a configuration, the user's head can be imaged even in a dark place. The imaging unit 32 may further include a thermocamera. This makes it possible to measure the surface temperature of the head of the user.

The monitoring unit 30 includes a monitoring control unit (not shown). The monitoring control unit transmits to the control unit 50 the information detected by the body motion detection unit 31 as body motion information, the image captured by the imaging unit 32 as image information, the information collected by the sound collecting unit 33 as sound information, the information measured by the blood oxygen measuring unit 34 as blood oxygen information, the information measured by the electrocardiogram measuring unit 35 as electrocardiogram information, and the information measured by the brain wave measuring unit 36 as brain wave information. The body motion information, the image information, the sound information, the blood oxygen information, the electrocardiographic information, and the brain wave information are examples of sleep information.

The control unit 50 includes a sleep information acquisition unit 51, an analysis unit 52, a transmission/reception unit 53, a classification unit 54, and a storage unit 55. The control unit 50 receives sleep information indicating the state of the user during sleep from the sleep monitoring capsule 1, and determines the presence or absence of an apnea state in which breathing stops during sleep of the user based on the sleep information.

The sleep information acquisition unit 51 continuously acquires the body motion information, the image information, the sound information, the blood oxygen information, the electrocardiographic information, and the brain wave information as sleep information from the monitoring unit 30 of the sleep monitoring capsule 1 at predetermined intervals, and sequentially stores them in the storage unit 55.

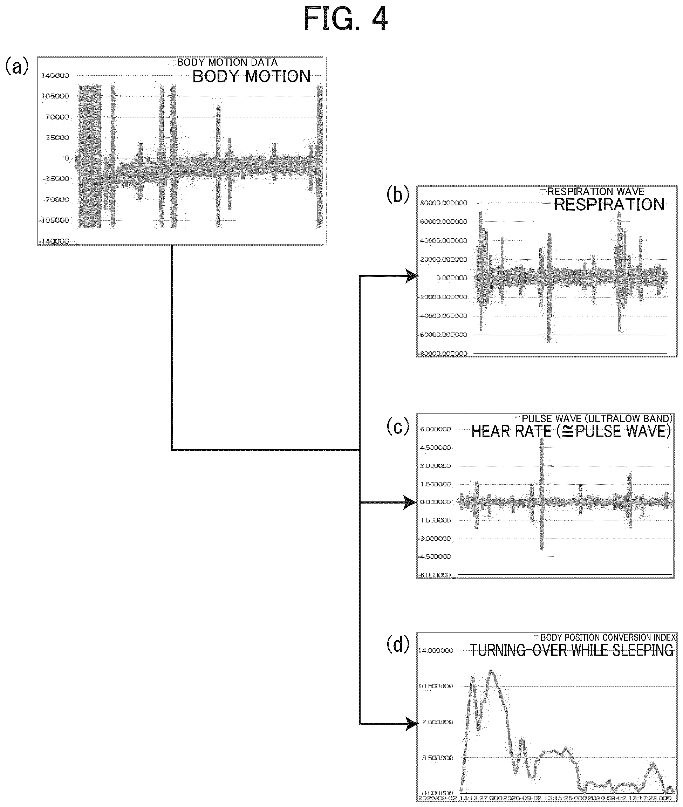

FIGS. 4 to 7 are diagrams each illustrating analysis by the analysis unit according to the embodiment of the present invention. The analysis unit 52 analyzes the body motion information acquired by the sleep information acquisition unit 51. Specifically, based on the continuous body motion information stored in the storage unit 55, the analysis unit 52 generates time-series data in which the continuous body motion information is expressed by waves having a magnitude of a value in the time series (FIG. 4A). Next, the analysis unit 52 decomposes the time series data of the body motion information into breathing time series data (FIG. 4B), heart rate time series data (FIG. 4C), and turning-over while sleep time series data (FIG. 4D) by a publicly-known method (for example, Fourier transform, AI (artificial intelligence) analysis, or the like).

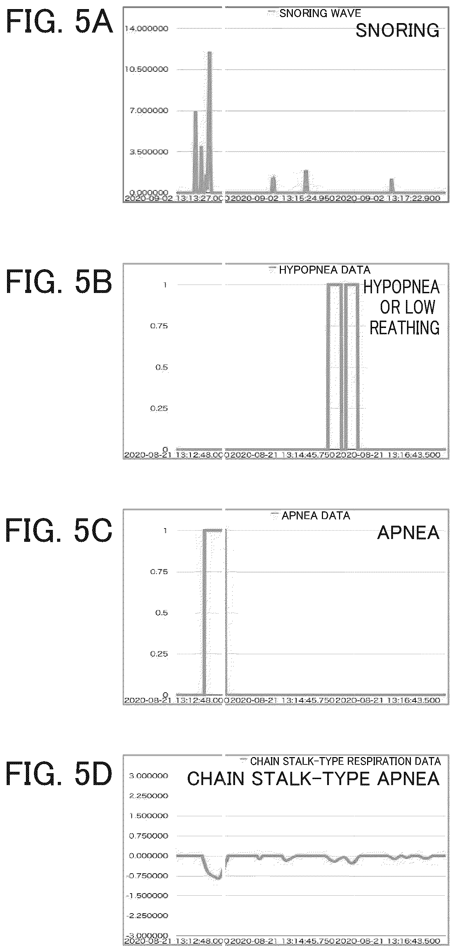

Furthermore, the analysis unit 52 performs frequency decomposition as shown in FIG. 5 by a well-known method (for example, Fourier transform, AI analysis, or the like) based on the time series data of breathing (FIG. 4B). By this frequency decomposition, for example, the analysis unit 52 determines that snoring is occurring in a case of the distribution shown in FIG. 5A, determines that hypopnea or low breathing is occurring in a case of the distribution shown in FIG. 1 5B, determines that apnea is occurring in a case of the distribution shown in FIG. 5C, and determines that chain stalk-type apnea is occurring in a case of the distribution shown in FIG. 5D.

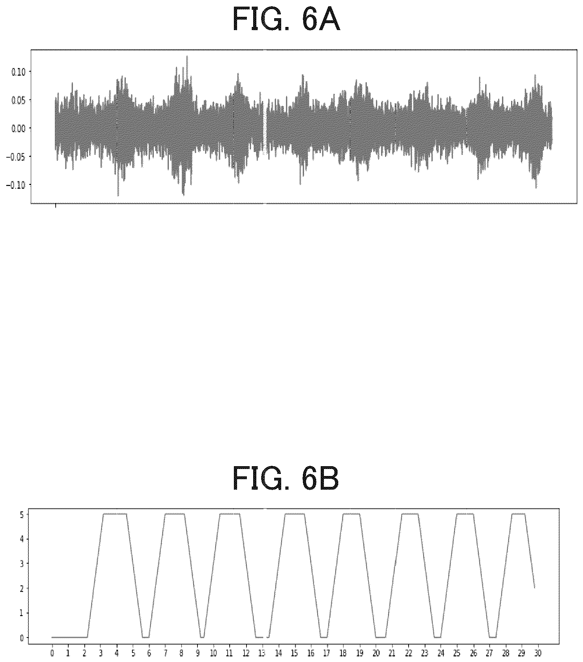

Furthermore, based on the continuous sound information stored in the storage unit 55, the analysis unit 52 generates time-series data in which the continuous sound information is expressed by waves having a magnitude of a value in the time series (FIG. 6A). Next, the analysis unit 52 performs frequency decomposition as shown in FIG. 6B by a publicly-known method (for example, Fourier transform, AI analysis, or the like) based on the time series data of the sound information. When the specific wavelength appears at a predetermined period, the analysis unit 52 determines that snoring is occurring.

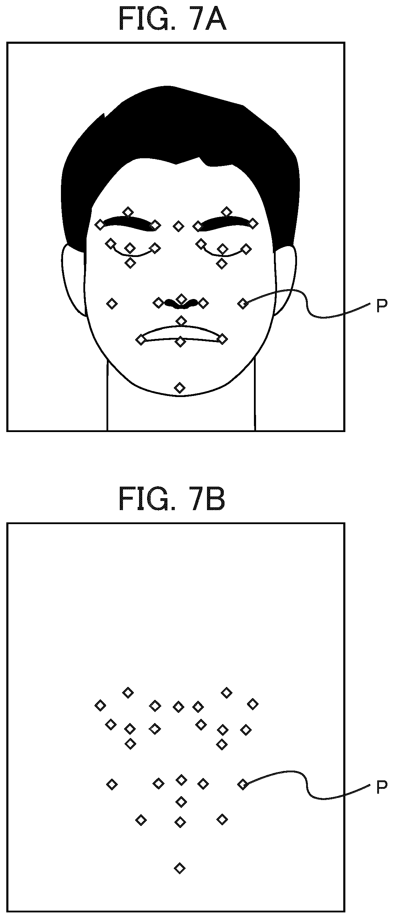

Furthermore, the analysis unit 52 may analyze the image information stored in the storage unit 55, set a point P (a rhombic portion in the example shown in FIG. 7) at a feature point (e.g., a contour portion of an eye, nose, mouth, etc.) of a person's face, compare a variation in time series of the point P with time series data based on body motion information (e.g., time series data of breathing shown in FIG. 4B) or data obtained by performing frequency decomposition on time series data of breathing (e.g., FIG. 5C), and determine whether or not breathing is performed. For example, in the distribution shown in FIG. 5C, when there is no variation in the point P at a time when there is no body motion based on breathing (a time when the value of the vertical axis is 0), the analysis unit 52 correctly determines that apnea is occurring.

Furthermore, the analysis unit 52 may delete a personally identifiable image (a face image shown in FIG. 7A) from the image information stored in the storage unit 55, and store only the point P in the storage unit 55 as shown in FIG. 7B. This makes it possible to provide a system in consideration of privacy of a user.

Furthermore, the analysis unit 52 may convert the continuous data of the blood oxygen information, the electrocardiogram information, and the brain wave information stored in the storage unit 55 into time-series data expressed by waves having a magnitude of a value in a time series, and may store the data in the storage unit 55 in a format that can be displayed on the terminal 200, for example.

With reference to FIG. 3 again, the transmission/reception unit 53 receives the user data (for example, data for identifying the user, a use history of the sleep monitoring capsule 1 of the user, and past sleep information) from the server 300 of the accommodation facility, associates the received data with the data based on the sleep information analyzed by the analysis unit 52, and transmits the data to the terminal 200. Furthermore, the transmission/reception unit 53 transmits data based on sleep information analyzed by the analysis unit 52 to the server 300 of the medical institution, receives a medical diagnosis result for the sleep information, associates the received diagnosis result with data based on the sleep information analyzed by the analysis unit 52, and transmits the data to the terminal 200.

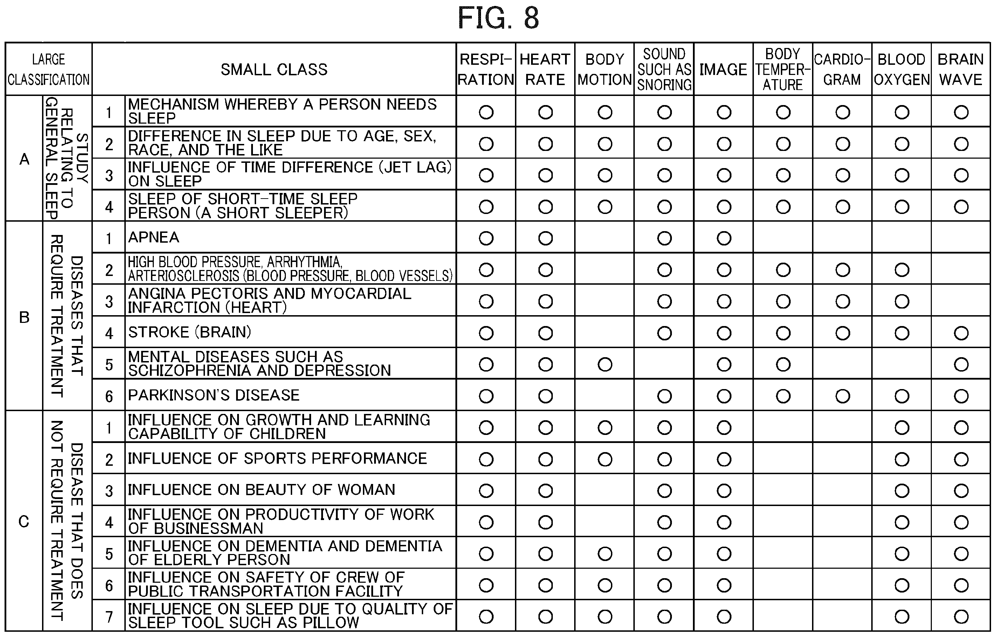

FIG. 8 is a diagram illustrating an outline of classification by the classification unit according to the embodiment of the present invention. The classification unit 54 stores the analysis result of the sleep information by the analysis unit 52 in the storage unit 55 as a block for each classification. More specifically, the classification unit 54 forms blocks of A (a study relating to general sleep), B (a disease requiring treatment), and C (a disease requiring no treatment) as large classifications. Then, the classification unit 54 further sub-classifies each large classification to form small-class blocks.

In FIG. 8, "respiratory" is time-series data of breathing (FIG. 4B), "heart rate" is time-series data of heart rate (FIG. 4C), "body motion" is time-series data of body motion (FIG. 4A), "sound such as snoring" is data obtained by performing frequency decomposition on time-series data of sound information (FIG. 6B), "image" is data obtained by image analysis of image information, "body temperature" is data measured by a thermocamera, "cardiogram" is time-series data of electrocardiographic information, "blood oxygen" is time series data of blood oxygen information, and "brain wave" is time series data of brain wave information. The classification unit 54 stores, in the storage unit 55, data of types corresponding to the small classifications (data rounded in FIG. 8) among these data as blocks.

The A-1 block is a block aimed at contributing to the study of a mechanism that a person needs sleep, and the data of the block makes it possible to verify the maximum mysteriousness of "why sleep is necessary" for an organism by using a specific and large amount of data.

The A-2 block is a block aimed at contributing to the study of difference in sleep due to age, sex, race, and the like, and it is possible to verify that the degree of sleep (time, depth, quality of sleep) of a person differs depending on attributes of the person such as age, race, and the like, and that the incidence rate of illness due to sleep is different depending on the data of the block.

The A-3 block is a block aimed at contributing to the study of the influence of the time difference (jet lag) on sleep, and it is possible to verify that the quality of sleep by the time difference due to airplane movement is degraded based on data of the block acquired from the user immediately after returning home.

The A-4 block is a block aimed at contributing to the study of sleep of a short-time sleep person (a short sleeper), and it is possible to verify what type of sleep is taken by a person of a short-time sleep person based on data of the block acquired from a user of the short-sleeper and whether the sleep can be applied to a general person.

The B-1 block is a block intended to contribute to diagnosis of apnea, and it is possible to accurately diagnose apnea by using data of the block.

The B-2 block is a block aimed at contributing to the diagnosis of high blood pressure, arrhythmia, arteriosclerosis (blood pressure, blood vessels), and, based on the data of this block, it is possible to diagnose diseases relating to blood pressure and pulsation wave such as high blood pressure, arrhythmia, and arteriosclerosis in advance by data relating to pulse disorders in addition to data relating to apnea.

The B-3 block is a block aimed at contributing to diagnosis of angina pectoris and myocardial infarction (heart), and, based on the data of the block, it is possible to diagnose diseases related to the heart, such as angina pectoris and myocardial infarction, by using data of heart rate disorder in addition to the data related to apnea.

The B-4 block is a block aimed at contributing to the diagnosis of a stroke (brain), and based on the data of this block, it is possible to diagnose a disease related to the brain such as a stroke in advance by the data related to brain waves in addition to the data related to apnea.

The B-5 block is a block aimed at contributing to the diagnosis of mental diseases such as schizophrenia and depression, and based on the data of this block, it is possible to perform more accurate diagnosis of mental diseases by adding "images" in addition to data for diagnosing the possibility of mental diseases from respiration and heart rate disorder.

The B-6 block is a block aimed at contributing to the diagnosis of Parkinson's disease, and, based on the data of this block, it is possible to find the possibility of Parkinson's disease when there is data indicating more specific sleep (body motion, abnormal body temperature, or the like) among users who see apnea or symptoms of psychological disease.

The C-1 block is a block aimed at contributing to the verification of the influence on the growth and learning capability of children, and based on the data of the block of the user who is a child, it is possible to verify that the quality and time of sleep have a large influence on the development of children and the development of the learning capability.

The C-2 block is a block aimed at contributing to the verification of the influence of the sports performance, and based on the data of the block of the user who is a sports player, it is possible to verify that the quality and time of sleep affects the growth of muscles of the sports player and the final performance, thereby enabling the sports player to find sleep optimally.

The C-3 block is a block aimed at contributing to the verification of the influence on the beauty of a woman, and based on data of the block of a user who is a woman, it is possible to verify that the quality and time of sleep affects the beauty of a woman (skin wrinkles, white hair, aging, and the like), thereby finding optimal sleep for the beauty of a woman.

The C-4 block is a block aimed at contributing to the verification of the influence on the productivity of work of a businessman, and based on data of the block of the user who is a businessman, it is possible to verify that the quality and time of sleep affects the productivity of a businessman who works for a long period of time every day, and in some cases, can lead to mental diseases, thereby enabling the businessman to find optimal sleep.

The C-5 block is a block aimed at contributing to the verification of the influence on dementia and dementia of an elderly person, and based on the data of the block of the user who is an elderly person, it is possible to verify that the quality and time of sleep affects the elderly person's dementia, and the like and proves and verifies that these diseases progress without proper sleep, thereby enabling the elderly person to find optimal sleep.

The C-6 block is a block aimed at contributing to the verification of the influence on the safety of a crew of a public transportation facility, and based on data of the block of a user who is a crew of the public transportation facility, it is possible to verify the degree of influence of irregular sleep on the operation of a crew of a public transportation facility (e.g., a conductor of the first train), thereby preventing the crew member from falling asleep or suffering from mental illness, as well as preventing major accidents.

The C-7 block is a block aimed at contributing to the verification of the influence on sleep due to quality of a sleep tool such as a pillow, and based on data of the block when a specific sleep tool is used, it is possible to verify with specific numerical values the quality of the specific sleep tool (pillow, mattress, etc.) and whether or not the quality of the specific sleep tool is suitable for each individual, thereby establishing a method of finding a sleep tool for providing optimal sleep.

According to the sleep monitoring capsule 1, by demarcating the sleep space in advance by the capsule 10, it is possible to suppress the influence of the environment outside the capsule 10 on the state of the user during sleep and the measurement data obtained by measuring the state of the user. Furthermore, by defining the position where the user is lying down by the sleep tool 20 in the capsule 10, it is possible to suppress the influence of the position where the user is lying down on the state of the user during sleep and the measurement data obtained by measuring the state of the user. Therefore, it is possible to provide a sleep monitoring capsule capable of improving the reliability of measurement data obtained by measuring the state of the user during sleep.

Furthermore, according to the sleep monitoring capsule 1, by providing the body motion detection unit 31 in contact with a predetermined position of the sleep tool 20 defining a position at which the user is lying down, it is possible to suppress variation of information detected by the body motion detection unit 31 due to the posture of the user during sleep and the position at which the body motion detection unit 31 is attached, and to make the conditions for obtaining information indicating the state of the user during sleep uniform.

Furthermore, according to the sleep monitoring capsule 1, the imaging unit 32 is fixed to the capsule 10 and captures an image of the head of the user lying down on the sleep tool 20 defining the position and the direction in which the user is lying down, whereby it is possible to suppress the variation of the image of the head of the user captured by the imaging unit 32 depending on the position and the direction in which the image is captured, and it is also possible to make the conditions for obtaining information indicating the state of the user during sleep uniform.

Furthermore, according to the sleep monitoring capsule 1, the sound collecting unit 33 collects sounds in the capsule 10 when the user is sleeping with the sleep tool 20, thereby suppressing the influence of sounds around the user and making it possible to make the conditions for obtaining information indicating the state of the user during sleep uniform.

Furthermore, according to the sleep monitoring system 100, since the presence or absence of the apnea state can be determined based on the sleep information acquired in a state in which the capsule 10 demarcates the sleep space in advance and the position where the user lies down is defined, it is possible to improve the accuracy of the determination.

The embodiments described above can be made as an invention as follows.

(1) A sleep monitoring capsule having a capsule that demarcates a sleep space for one person for monitoring a sleep state of a user sleeping in the capsule, the sleep monitoring capsule comprising a sleep tool that is provided in the capsule and on which a user lies down, and a monitoring unit that is provided in the capsule and monitors a state of the user sleeping with the sleep tool.

In the invention of (1), a sleep monitoring capsule having a capsule for demarcating a sleep space for one person and monitoring a sleep state of a user sleeping in the capsule includes a sleep tool and a monitoring unit. The sleep tool is provided in the capsule and the user lies down on the sleep tool. The monitoring unit is provided in the capsule and monitors the state of the user during sleep with the sleep tool.

According to the invention of (1), the sleep tool on which the user is lying down is provided in the capsule that demarcates the sleep space for one person, and the state of the user during sleep with the sleep tool is monitored.

Thus, by demarcating the sleep space by the capsule in advance, it is possible to suppress the influence of the environment outside the capsule on the state of the user during sleep and the measurement data obtained by measuring the state of the user. Furthermore, in this capsule, by defining the position at which the user is lying down by the sleep tool, it is possible to suppress the influence of the position at which the user is lying down on the state of the user during sleep and the measurement data obtained by measuring the state of the user. Therefore, it is possible to provide a sleep monitoring capsule capable of improving the reliability of measurement data obtained by measuring the state of the user during sleep.

(2) The sleep monitoring capsule according to (1), wherein the monitoring unit is provided in contact with a predetermined position of the sleep tool, and includes a body motion detection unit for detecting a movement of a body of a user transmitted to the sleep tool.

According to the invention of (2), the monitoring unit is provided in contact with a predetermined position of the sleep tool, and includes a body motion detection unit that detects a movement of the body of the user transmitted to the sleep tool.

Here, when the body motion detection unit is directly attached to the body of the user, the information detected by the body motion detection unit varies depending on the posture of the user during sleep and the position where the body motion detection unit is attached.

According to the invention of (2), by providing the body motion detection unit so as to contact a predetermined position of the sleep tool that defines a position at which the user lies down, it is possible to suppress a variation in information detected by the body motion detection unit due to a posture of the user during sleep or a position at which the body motion detection unit is attached, and to make a condition for acquiring information indicating a state of the user during sleep uniform. Therefore, it is possible to provide a sleep monitoring capsule capable of improving the reliability of measurement data obtained by measuring the state of the user during sleep.

(3) The sleep monitoring capsule according to (1) or (2), wherein the sleep tool is provided at a predetermined position of the capsule and defines an orientation in which a user lies down, and the monitoring unit is fixed to the capsule, and further includes an imaging unit for imaging at least the head of a user lying down on the sleep tool.

According to the invention of (3), the sleep tool is provided at a predetermined position of the capsule, and defines a direction in which the user is lying down. The monitoring unit is fixed to the capsule, and further includes an imaging unit for imaging at least the head of the user lying down on the sleep tool.

Here, when the imaging unit for imaging the head of the user during sleep is installed, the image of the head of the user captured by the imaging unit varies depending on the position and the direction of the imaging, and even if the analysis is performed based on such an image, the analysis result varies, so that the reliability of the analysis result may decrease.

According to the invention of (3), the imaging unit is fixed to the capsule and images the head of the user lying down in the sleep tool that defines the position and the direction in which the user is lying down, whereby it is possible to suppress the variation in the image of the head of the user captured by the imaging unit depending on the position and the direction of the imaging, and it is possible to make the condition for obtaining information indicating the state of the user during sleep uniform. Therefore, it is possible to provide a sleep monitoring capsule capable of improving the reliability of measurement data obtained by measuring the state of the user during sleep.

(4) The sleep monitoring capsule according to any one of (1) to (3), wherein the monitoring unit further includes a sound collecting unit for collecting sound in the capsule while the user is sleeping with the sleep tool.

In the invention of (4), the monitoring unit further includes a sound collecting unit for collecting sound in the capsule while the user is sleeping with the sleep tool.

Here, when sounds generated by a user during sleep who sleeps in an open environment are collected, not only sounds generated by the user but also sounds around the user are collected, and even when the analysis is performed based on sounds including sounds around the user, the analysis cannot be accurately performed and reliability of the analysis result may be lowered.

According to the invention of (4), the sound collecting unit collects the sound in the capsule while the user is sleeping with the sleep tool, whereby it is possible to suppress the influence of the sound around the user, and it is also possible to make the condition for obtaining the information indicating the state of the user during sleep uniform. Therefore, it is possible to provide a sleep monitoring capsule capable of improving the reliability of measurement data obtained by measuring the state of the user during sleep.

(5) A sleep monitoring system comprising a sleep monitoring capsule according to any one of (1) to (4); and a controller that receives sleep information indicating a state of a user during sleep from the monitoring unit, and determines presence or absence of an apnea state in which breathing stops during sleep of the user based on the sleep information.

In the invention of (5), the sleep monitoring system includes the sleep monitoring capsule according to any one of (1) to (4), and a controller. The controller receives sleep information indicating a state of the user during sleep from the monitoring unit, and determines presence or absence of an apnea state in which breathing stops during sleep of the user based on the sleep information.

According to the invention of (5), it is possible to determines presence or absence of an apnea state in which breathing stops during sleep of the user based on sleep information received from the monitoring unit of the sleep monitoring capsule according to any one of (1) to (4).

Thus, since the presence or absence of the apnea state can be determined based on the sleep information acquired in a state in which the sleep space is demarcated by the capsule in advance and the position where the user is lying down is defined, it is possible to improve the accuracy of the determination.

According to the above invention, it is possible to provide a sleep monitoring capsule and a sleep monitoring system capable of improving reliability of measurement data obtained by measuring a state of a user during sleep.

### Application Example

### Configuration of Sleep Monitoring Capsule according to Application Example

Next, an application example of the embodiment of the present invention will be described. In the following description, the same components as those in the above embodiment are denoted by the same reference numerals, and the description thereof will be omitted or simplified.

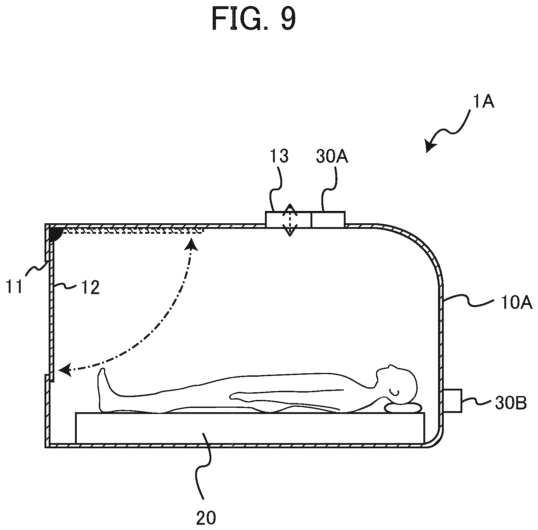

FIG. 9 is a diagram illustrating an outline of a sleep monitoring capsule according to an application example of the embodiment of the present invention. A sleep monitoring capsule 1A according to the application example is mainly different from the sleep monitoring capsule 1 according to the embodiment in that the sleep monitoring capsule 1A includes a door 12 and a ventilation unit 13.

The capsule 10A of the application example is the same as the capsule 10 according to the embodiment except for the difference therebetween which will be described below. The capsule 10A is preferably formed to have an outer dimension including a frame (not shown) for holding the outer dimension of the capsule 10A in accordance with the inner dimension of the export container (for example, a 20-feet container (length 6,007 mm × width 2,328 mm × height 2,178 mm)). For example, the outer dimension of the capsule 10A is preferably 2,300 mm in the depth (the direction from the user's feet to the head), which is a dimension slightly smaller than the inner width dimension of the 20-feet container), and the width dimension (the direction orthogonal to the direction from the user's feet to the head) is also preferably slightly smaller than the inner length dimension of the 20-feet container by multiplying the width by an integer. The outer dimension of the capsule 10A is preferably slightly smaller than the inner height dimension (2,061 mm) of the entrance of the export container by multiplying the height dimension by an integer. By setting the outer dimension of the capsule 10A to such a dimension, it is possible to improve the transport efficiency in a case of exporting the capsule 10A.

The door 12 has a lid shape capable of opening and closing an opening 11 serving as an entrance/exit of the capsule 10A from an inner side of the capsule 10A. The door 12 is preferably a sealed type that closes off the inside and outside of the opening 11. The door 12 may have any configuration such as a shutter as long as the door 12 can close the inside and the outside of the opening 11.

The ventilation unit 13 ventilates the inside of the capsule 10A. The ventilation unit 13 includes a ventilation port which is relatively low in noise level and communicates with the outside of the capsule 10A, and an intake/exhaust port of an air conditioning facility.

The sleep monitoring capsule 1A includes a monitoring unit 30A similar to the monitoring unit 30 of the sleep monitoring capsule 1 according to the embodiment, and a capsule control unit 30B that transmits sleep information indicating the state of the user during sleep acquired by the monitoring unit 30A to a control unit 150 described later.

The monitoring unit 30A monitors the state of the user during sleep in the capsule 10A. The monitoring unit 30A includes an acquisition unit (a camera, a microphone, a sensor, and the like) similar to the monitoring unit 30 (the body motion detection unit 31, the imaging unit 32, the sound collecting unit 33, the blood oxygen measuring unit 34, the electrocardiogram measuring unit 35, the brain wave measuring unit 36, and the like) of the sleep monitoring capsule 1 according to the embodiment. For example, a camera or a microphone corresponding to the imaging unit 32 or the sound collecting unit 33 may be provided on the outside of the capsule 10A via a shielding plate (e.g., a beam splitter or the like) with which the inside of the capsule 10A is visible from the outside of the capsule 10A and the outside is not visible from the inside. This makes it possible to monitor the state of the user during sleep while reducing the consciousness that the user is being monitored.

In addition to the above detection unit, the monitoring unit 30A may include another detection unit capable of acquiring information necessary for monitoring the state of saliva, urine, sweat, DNA, etc., in the user. This further improves the accuracy of analysis.

The capsule control unit 30B controls the monitoring unit 30A, and transmits the sleep information acquired by the monitoring unit 30A to the control unit 150 described later. The capsule control unit 30B may also control illumination for illuminating the inside of the capsule 10A or air conditioning equipment for air conditioning the inside of the capsule 10A.

Furthermore, the capsule control unit 30B may acquire body information (e.g., height, weight, etc.) relating to the body of the user based on an operation of the user, and transmit such body information to the control unit 150.

### Overall Configuration of Sleep Monitoring System according to Application Example

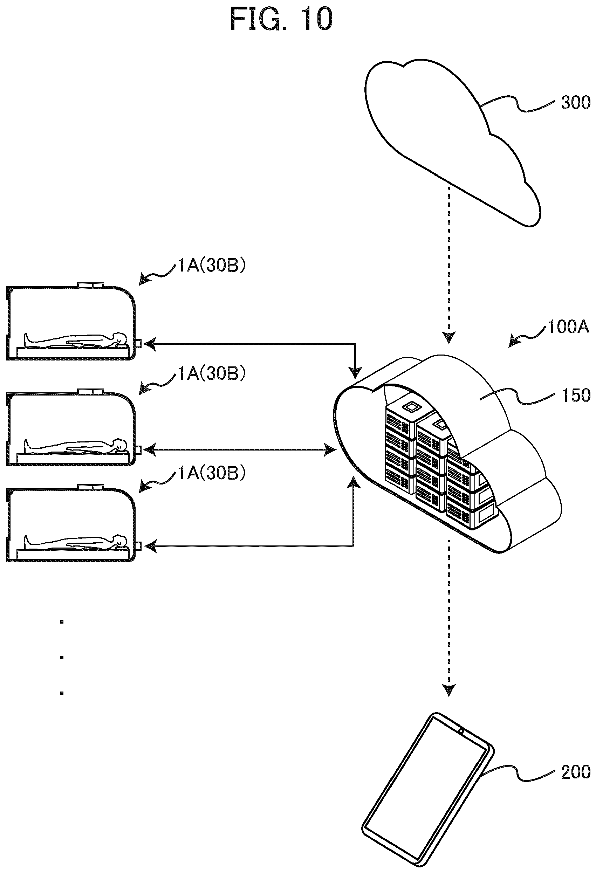

FIG. 10 is a diagram illustrating an outline of a sleep monitoring system according to an application example of the embodiment of the present invention. The sleep monitoring system 100A includes a sleep monitoring capsule 1A and a control unit 150 connected to the sleep monitoring capsule 1A via a network.

In this application example, the control unit 150 is configured by, for example, a cloud server connected via the Internet to the capsule control unit 30B that controls the monitoring unit 30A of the sleep monitoring capsule 1A; however, the present invention is not limited thereto, and may be, for example, an apparatus (computer) installed in an accommodation facility (e.g., capsule hotel or the like) in which the sleep monitoring capsule 1A is installed. In this case, this device may function as the capsule control unit 30B.

Furthermore, a plurality of sleep monitoring capsules 1A can be connected to the control unit 150. The control unit 150 can be connected to a terminal 200 outside the system and a server 300 outside the system via a network.

The terminal 200 is, for example, a smartphone, a tablet, or a personal computer, and is a terminal operated by a user of the sleep monitoring capsule 1A. The user can receive the sleep information in the sleep monitoring capsule 1A, the determination result based on the sleep information, and the like at the terminal 200, and confirm the information. Furthermore, the terminal 200 may acquire body information (e.g., height, weight, etc.) relating to the body of the user based on an operation of the user, and transmit such body information to the control unit 150.

The server 300 is, for example, a server of an accommodation facility in which the sleep monitoring capsules 1 are installed. Examples of the server 300 include a server for storing customer data of the accommodation facility and a server of a medical institution in which medical diagnosis of sleep information can be performed such as a medical institution and a university, or the like. Furthermore, the server 300 may acquire body information (e.g., height, weight, etc.) relating to the body of the user and transmit such body information to the control unit 150.

### Overall Configuration of Sleep Monitoring System according to Application Example

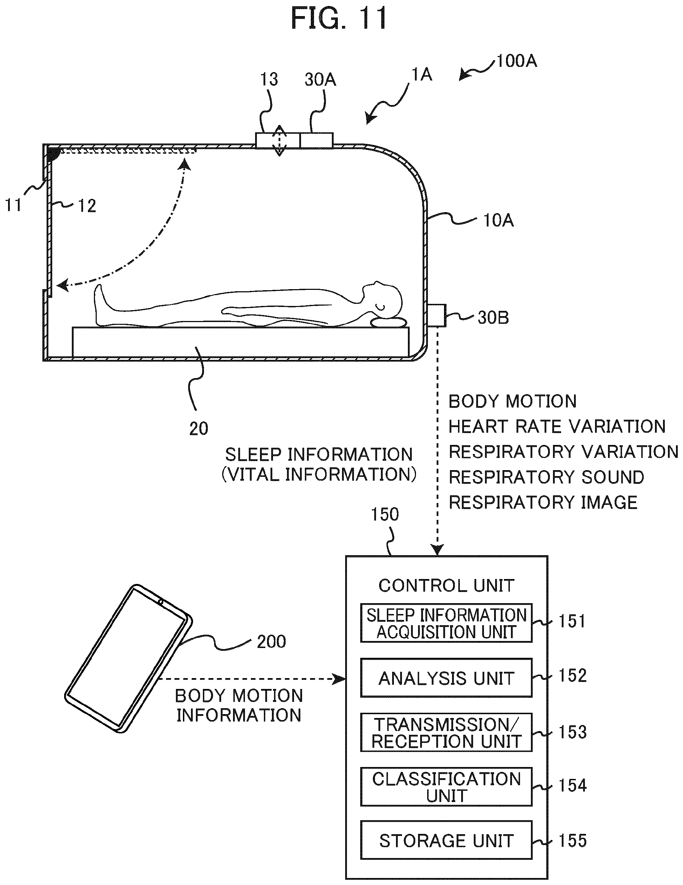

FIG. 11 is a diagram showing a functional configuration of a sleep monitoring system according to an application example of the embodiment of the present invention. In the sleep monitoring system 100A, the sleep monitoring capsule 1A includes, as a monitoring unit 30A, an acquisition unit (a camera, a microphone, a sensor, and the like) similar to the monitoring unit 30 (the body motion detection unit 31, the imaging unit 32, the sound collecting unit 33, the blood oxygen measuring unit 34, the electrocardiogram measuring unit 35, the brain wave measuring unit 36, and the like.) of the sleep monitoring capsule 1 according to the embodiment. Furthermore, the monitoring unit 30 may include a sensor for detecting a change in the body pressure distribution of the whole body or a 3D distance measuring sensor.

The sleep monitoring capsule 1A transmits information indicating body motion, heart rate variation, respiratory variation, respiratory sound, respiratory image, and the like, which are examples of sleep information (vital information) acquired by the monitoring unit 30A, to the control unit 150 by the capsule control unit 30B.

The control unit 150 includes an acquisition unit 151, an analysis unit 152, a transmission/reception unit 153, a classification unit 154, and a storage unit 155. The control unit 150 receives sleep information indicating the state of the user during sleep from the sleep monitoring capsule 1A, receives, for example, physical information (e.g., height, weight, etc.) regarding the body of the user from the terminal 200, and determines the state during sleep of the user during sleep of the user based on the sleep information and the physical information.

The acquisition unit 151 continuously acquires the body motion information, the image information, the sound information, the blood oxygen information, the electrocardiographic information, and the brain wave information at predetermined intervals as sleep information (vital information during sleep) acquired by the monitoring unit 30A from the capsule control unit 30B of the sleep monitoring capsule 1A, and sequentially stores them in the storage unit 155. The acquisition unit 151 receives the body information (e.g., height, weight, etc.) relating to the body of the user from the capsule control unit 30B or the terminal 200, and stores the body information in the storage unit 155.

The analysis unit 152 has the following functions in addition to the same functions as the analysis unit 52 according to the embodiment. The analysis unit 152 analyzes the state of the user during sleep by mutually complementing a plurality of pieces of sleep information detected by the plurality of detection units included in the monitoring unit 30A of the sleep monitoring capsule 1A.

More specifically, the analysis unit 152 performs time-frequency conversion on the sleep information acquired by each of the plurality of detection units, calculates the respective information bases, performs causal relationship analysis and mutual information amount network analysis between the information bases, and performs mutual complementation based on a change in mutual information amount at each base node.

Furthermore, the analysis unit 152 performs correction based on the body information on the sleep information, and analyzes the state of the user during sleep.

More specifically, the analysis unit 152 calculates a bias based on the body information (e.g., height, weight, etc.), and corrects the information bases calculated from the sleep information with this bias.

The analysis unit 152 can also simulate a change in the state of the body based on information relating to the body motion of the user acquired by the monitoring unit 30 (for example, a sensor for detecting a change in the body pressure distribution of the whole body, a 3D distance measurement sensor, or the like). By simulating such a change in the state of the body, it is possible to estimate a change in the body motion in three-dimensional data based on partial data of the body.

The transmission/reception unit 153 receives the user data (for example, data for identifying the user, a use history of the sleep monitoring capsule 1 of the user, and past sleep information) from the server 300 of the accommodation facility, associates the data with the data based on the sleep information analyzed by the analysis unit 152, and transmits the data to the terminal 200. Furthermore, the transmission/reception unit 53 transmits data based on the sleep information analyzed by the analysis unit 52 to the server 300 of the medical institution, receives a medical diagnosis result for the sleep information, associates the data based on the sleep information analyzed by the analysis unit 152 with the received diagnosis result, and transmits the data to the terminal 200.

The classification unit 154 has the following functions in addition to the same functions as the classification unit 54 according to the embodiment. The classification unit 154 classifies the state of the user during sleep analyzed by the analysis unit 152 for each of a plurality of kinds of diseases that are closely related to sleep. For example, diseases that are closely related to sleep include high blood pressure, sleep apnea syndrome, REM sleep behavior disorder, PLMD (periodic limb movement disorder), frequent urination, predisposition toward allergies, and the like, and cardiac muscle cerebral infarction, Parkinson's disease, and depression insomnia induced from these diseases. The information indicating the state of the user during sleep for diagnosing these diseases is different from each other. The classification unit 154 classifies the information indicating the state of the user during sleep, which is analyzed by the analysis unit 152, into those necessary for diagnosing each disease for every disease.

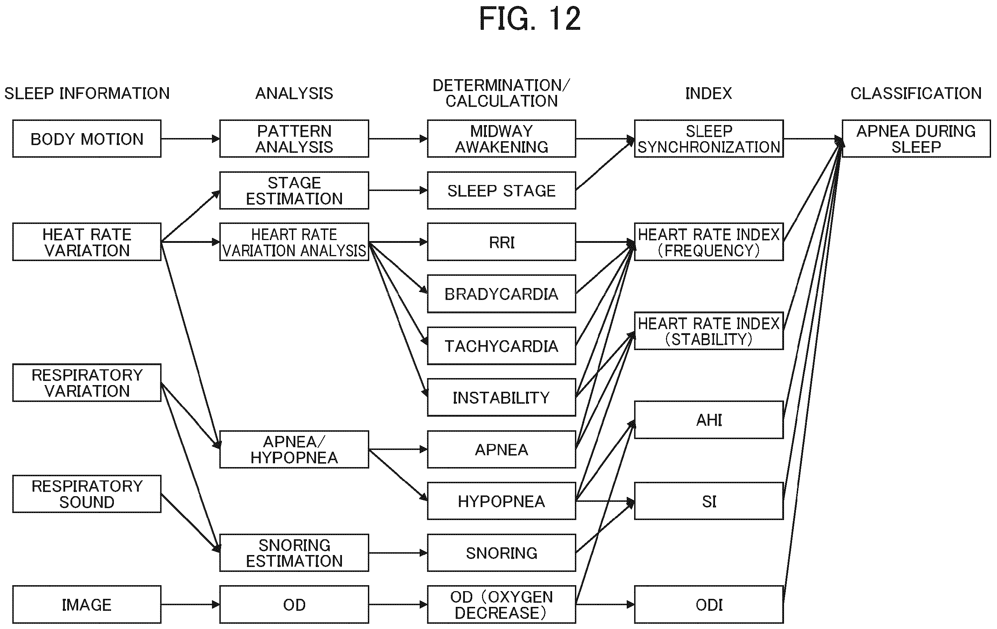

Next, functions of the analysis unit 152 and the classification unit 154 will be specifically described. FIG. 12 is a diagram illustrating functions of an analysis unit and a classification unit according to an application example of the embodiment of the present invention. The example shown in FIG. 12 shows the flow of processing in which the analysis unit 152 analyzes information indicating the state of the user during sleep classified into sleep apnea syndrome by the classification unit 154.

In the example shown in FIG. 12, the acquisition unit 151 acquires information indicating body motion, heart rate variation, respiratory variation, respiratory sound, and respiratory image as sleep information from the sleep monitoring capsule 1A.

The analysis unit 152 analyzes the sleeping state of the user based on the information acquired by the acquisition unit 151. Specifically, the analysis unit 152 analyzes the sleep pattern based on the information indicating a body motion. Furthermore, the analysis unit 152 estimates a stage indicating the depth of sleep based on information indicating a heart rate variation. The analysis unit 152 also analyzes the heart rate variation based on the information indicating the heart rate variation. Furthermore, the analysis unit 152 estimates the presence or absence of apnea or hypopnea (or low breathing) based on the information indicating the heart rate variation and a respiratory variation. Furthermore, the analysis unit 152 estimates the presence or absence of snoring based on the information indicating the respiratory variation and respiratory sounds. The analysis unit 152 estimates OD (Oxygen Desaturation) based on the information indicating a respiratory image.

Next, the analysis unit 152 determines whether or not there is a midway awakening from the analysis result of the sleep pattern. The analysis unit 152 also determines the sleep stage based on the estimation result of the stage. The analysis unit 152 calculates an RRI (R-R Interval) variation based on the analysis result of the heart rate variation. Furthermore, the analysis unit 152 determines the presence or absence of bradycardia and tachycardia and the instability of the heart rate based on the analysis result of the heart rate variation. The analysis unit 152 also determines the presence or absence of apnea and hypopnea based on the estimation result of the presence or absence of apnea and hypopnea. Furthermore, the analysis unit 152 determines the presence or absence of snoring based on the estimation result of the presence or absence of snoring. The analysis unit 152 also determines the degree of OD based on the estimation result of OD.

Next, the analysis unit 152 sets the determination/calculation result as information indicating a predetermined index. Specifically, the analysis unit 152 generates information indicating sleep synchronization based on the presence or absence of middle awakening and the determination result of the sleep stage. In addition, the analysis unit 152 generates information indicating the heart rate index (frequency) based on the calculation result of the RRI variation, the determination result of the presence or absence of bradycardia and tachycardia, the determination result of the instability of the heart rate, and the determination result of the presence or absence of apnea and hypopnea. The analysis unit 152 also generates information indicating a heart rate index (stability) based on the determination result of the instability of the heart rate and the determination result of the presence or absence of apnea and hypopnea. Furthermore, the analysis unit 152 generates information indicating AHI (Apnea Hypopnea Index) based on the determination result of the presence or absence of hypopnea and the determination result of the degree of OD. The analysis unit 152 also generates information indicating SI (Snore Index: Snoring Index (AHI: Indicator for snoring for apnea hypopnea index)) based on the determination result of the presence or absence of hypopnea and the determination result of the presence or absence of snoring. The analysis unit 152 generates information indicating ODI (Oxygen Desaturation Index: Oxygen saturation decrease index) based on the determination result of the OD degree.

Then, the classification unit 154 classifies the information indicating sleep synchronization, the information indicating the heart rate index (frequency), the information indicating the heart rate index (stability), the information indicating the AHI, the information indicating the SI, and the information indicating the ODI generated by the analysis unit 152 into the information relating to the sleep apnea syndrome (information necessary for diagnosing the sleep apnea syndrome) among a plurality of types of diseases that are closely related to sleep.

The transmission/reception unit 153 transmits the information classified by the classification unit 154 into the information relating to the sleep apnea syndrome (information necessary for diagnosis of the sleep apnea syndrome) to the server 300 of a medical institution such as a doctor who diagnoses the sleep apnea syndrome. Furthermore, the transmission/reception unit 153 may receive the diagnosis result of the sleep apnea syndrome from the server 300 of the medical institution. Furthermore, the diagnosis result of the sleep apnea syndrome may be transmitted from the server 300 of the medical institution to the terminal 200 of the user.

The functional configuration of the system in the above-described embodiment and application example is merely an example, and one functional block (database and function processing unit) may be divided or a plurality of functional blocks may be collectively configured as one functional block. Each function processing unit is implemented by a computer program (for example, main software, applications that cause a CPU (Central Processing Unit) to execute the above-described various processes, or the like) executed by the CPU when the CPU built in the apparatus or the terminal reads a computer program (for example, the main software, an application that causes the CPU to execute the above-described various processes, or the like) stored in a storage device (storage unit) such as ROM (Read Only Memory), flash memory, a SSD (Solid State Drive), or a hard disk. That is, each function processing unit is implemented by the computer program reading and writing necessary data such as a table from a database (DB: Data Base) stored in a storage device or a storage area in memory, and controlling related hardware (for example, an input/output device, a display device, and a communication device (e.g., a Wi-Fi compatible device conforming to IEEE802.11, a wireless device conforming to International Telecommunication Union standards such as a third generation, a fourth generation, and a fifth generation mobile communication system, etc.)) in some cases. Furthermore, the database (DB) in the embodiment of the present invention may be a commercial database, but simply indicates a collection of tables and files, and the internal structure of the database itself is not limited.

According to the sleep monitoring capsule 1A of this application example, by dividing the sleep space in advance by the capsule 10A, it is possible to suppress the influence of the environment outside the capsule 10A on the state of the user during sleep and the measurement data obtained by measuring the state of the user. Therefore, it is possible to provide a sleep monitoring capsule capable of improving the reliability of measurement data obtained by measuring the state of the user during sleep.

Furthermore, according to the sleep monitoring capsule 1A, by closing the opening 11 of the capsule 10A from the inner side of the capsule 10A with the door 12, it is possible to improve the sound insulating property of the inside and outside of the capsule 10A. Thus, it is possible to suppress noise mixed in the measurement data obtained by measuring the state of the user during sleep. Furthermore, since the ventilation unit 13 is provided, even when the airtightness of the door is improved in order to improve the sound insulating property of the inside and outside of the capsule 10A, it is still possible to keep the environment such as the oxygen concentration and the temperature in the capsule 10A constant by ventilation by the ventilation unit 13. Therefore, it is possible to provide a sleep monitoring capsule capable of improving the reliability of measurement data obtained by measuring the state of the user during sleep.

Furthermore, according to the sleep monitoring system 100A of the application example, since the capsule 10A demarcates the sleep space in advance, and the state of the user during sleep is analyzed based on the sleep information of the user during sleep in the closed sleep space, it is possible to improve the accuracy of the analysis.

Furthermore, according to the sleep monitoring system 100A, by classifying the state of the user during sleep, which is an analysis result, for each disease that is closely related to sleep, it is possible to more efficiently operate the analysis result and improve the accuracy of the diagnosis of the professional practitioner in the future.

Furthermore, according to the sleep monitoring system 100A, since the plurality of pieces of sleep information detected by the plurality of detection unit are mutually complementing and the state of the user during sleep is analyzed, it is possible to analyze the plurality of pieces of sleep information based on mutual numerical changes, and the accuracy of analysis is improved.

Furthermore, according to the sleep monitoring system 100A, since the correction based on the body information relating to the body of the user is performed and the state of the user during sleep is analyzed, analysis according to the user becomes possible, and the accuracy of analysis is improved.

However, it is needless to say that the technical scope of the present invention is not limited to the above embodiments. It is apparent to those skilled in the art that various changes and modifications can be made to the above-described embodiments and application examples. It is also apparent from the claims that embodiments to which such changes or improvements are added can be included in the technical scope of the present invention. Unless contradictory, the embodiments and application examples can be combined with each other in any configuration. Although the sleep monitoring capsule and the sleep monitoring system have been described as the invention of the present invention in the above-described embodiments, the present invention can be regarded as a method executed by the sleep monitoring system and a program for causing the sleep monitoring system to function as various units.

### EXPLANATION OF REFERENCE NUMERALS

1A Sleep Monitoring Capsule , 10A Capsule, 11 Opening, 12 Door, 13 Ventilation Unit, 30A Monitoring Unit, 30B Capsule Control Unit, 100A Sleep Monitoring System , 150 Control Unit, 151 Obtaining Method , 152 Analysis Unit, 153 Transmission/Reception unit, 154 Classification Unit, 155 Storage unit, 200 Terminal, 300 Server

## Claims

1. A sleep monitoring capsule having a capsule that demarcates a sleep space for one person for monitoring a sleep state of a user sleeping in the capsule, the sleep monitoring capsule comprising:
a monitoring unit provided in the capsule, disposed at a position apart from the user, and configured to monitor a state of the user during sleep in the capsule,
wherein the monitoring unit includes a plurality of detection means for detecting the state of the user during sleep, and
wherein the detection means includes a body motion detection means disposed so as to be in contact with a predetermined position of bedding that defines a lying position of the user in the capsule.

2. The sleep monitoring capsule according to claim 1, wherein the capsule includes:
a door that is capable of opening and closing an entrance/exit from an inner side of the capsule, and
a ventilation unit that ventilates inside of the capsule.

3. A sleep monitoring system comprising:
a sleep monitoring capsule according to claim 1 or 2; and
a controller that determines a state of a user during sleep, wherein the controller includes:
an acquisition unit that acquires sleep information indicating the state of the user during sleep from the monitoring unit, and
an analysis unit that analyzes the state of the user during sleep based on the sleep information.

4. The sleep monitoring system according to claim **3,** wherein the controller further includes a classification unit that classifies the state of the user during sleep analyzed by the analysis unit for each disease that is closely related to sleep.

5. The sleep monitoring system according to claim 3 or **4,** wherein
the monitoring unit includes a plurality of detection units that each detect the state of the user during sleep, and
the analysis unit analyzes the state of the user during sleep by mutually complementing a plurality of pieces of sleep information detected by the plurality of detection units.

6. The sleep monitoring system according to any one of claims 3 to **5,** wherein
the monitoring unit acquires body information relating to a body of the user,
the acquisition unit acquires the body information from the monitoring unit, and
the analysis unit performs correction based on the body information and analyzes the state of the user during sleep.

## Patentansprüche

1. Schlafüberwachungskapsel mit einer Kapsel, die einen Schlafbereich für eine Person abgrenzt, um den Schlafzustand eines in der Kapsel schlafenden Benutzers zu überwachen, wobei die Schlafüberwachungskapsel umfasst:
eine Überwachungseinheit, die in der Kapsel vorgesehen ist, an einer vom Benutzer entfernten Position angeordnet ist und dazu eingerichtet ist, einen Zustand des Benutzers während des Schlafens in der Kapsel zu überwachen,
wobei die Überwachungseinheit mehrere Erfassungsmittel zum Erfassen des Zustands des Benutzers während des Schlafs umfasst, und
wobei die Erfassungsvorrichtung eine Körperbewegungserfassungsvorrichtung umfasst, die so angeordnet ist, dass sie mit einer vorbestimmten Position der Bettwäsche in Kontakt steht, die eine Liegeposition des Benutzers in der Kapsel definiert.

2. Die Schlafüberwachungskapsel gemäß Anspruch 1, wobei die Kapsel umfasst:
eine Tür, die einen Eingang/Ausgang von einer Innenseite der Kapsel aus öffnen und schließen kann, und
eine Belüftungseinheit, die das Innere der Kapsel belüftet.

3. Ein Schlafüberwachungssystem, das Folgendes umfasst:
eine Schlafüberwachungskapsel gemäß Anspruch 1 oder 2; und eine Steuereinheit, die einen Zustand eines Benutzers während des Schlafs bestimmt, wobei die Steuereinheit umfasst:
eine Erfassungseinheit, die Schlafdaten, die den Zustand des Benutzers während des Schlafs anzeigen, von der Überwachungseinheit ermittelt, und
eine Analyseeinheit, die den Zustand des Benutzers während des Schlafs auf der Grundlage der Schlafinformationen analysiert.

4. Schlafüberwachungssystem nach Anspruch 3, wobei die Steuereinheit ferner eine Klassifizierungseinheit umfasst, die den von der Analyseeinheit analysierten Zustand des Benutzers während des Schlafs für jede Krankheit klassifiziert, die mit Schlaf eng verwandt ist.

5. Schlafüberwachungssystem nach Anspruch 3 oder 4, wobei
die Überwachungseinheit mehrere Erfassungseinheiten aufweist, die jeweils den Zustand des Benutzers während des Schlafs erfassen, und die Analyseeinheit den Zustand des Benutzers während des Schlafs analysiert, indem sie mehrere von den mehreren Erfassungseinheiten erfasste Schlafdaten gegenseitig ergänzt.

6. Schlafüberwachungssystem nach einem der Ansprüche 3 bis 5, wobei
die Überwachungseinheit Körperinformationen in Bezug auf den Körper des Benutzers ermittelt,
die Erfassungseinheit die Körperdaten von der Überwachungseinheit ermittelt, und
die Analyseeinheit auf der Grundlage der Körperinformationen Korrekturen durchführt und den Zustand des Benutzers während des Schlafs analysiert.

## Revendications

1. Capsule de surveillance du sommeil présentant une capsule qui définit un espace de sommeil pour une personne pour la surveillance d'un état de sommeil d'un utilisateur dormant dans la capsule, la capsule de surveillance du sommeil comprenant :
une unité de surveillance prévue dans la capsule, disposée à une position à l'écart de l'utilisateur, et configurée pour surveiller un état de l'utilisateur pendant le sommeil dans la capsule, dans laquelle l'unité de surveillance inclut une pluralité de moyens de détection pour détecter l'état de l'utilisateur pendant le sommeil, et
dans laquelle les moyens de détection incluent un moyen de détection de mouvements du corps disposé de manière à être en contact avec une position prédéterminée de la literie qui définit une position allongée de l'utilisateur dans la capsule.

2. Capsule de surveillance du sommeil selon la revendication 1, dans laquelle la capsule inclut :
une porte qui est apte à ouvrir et fermer une entrée/sortie depuis un côté interne de la capsule, et
une unité de ventilation qui ventile l'intérieur de la capsule.

3. Système de surveillance du sommeil comprenant :
une capsule de surveillance du sommeil selon la revendication 1 ou la revendication 2 ; et
un dispositif de commande qui détermine un état d'un utilisateur pendant le sommeil, dans lequel le dispositif de commande inclut :
une unité d'acquisition qui acquiert des informations de sommeil indiquant l'état de l'utilisateur pendant le sommeil auprès de l'unité de surveillance, et
une unité d'analyse qui analyse l'état de l'utilisateur pendant le sommeil sur la base des informations de sommeil.

4. Système de surveillance du sommeil selon la revendication 3, dans lequel le dispositif de commande inclut en outre une unité de classification qui classe l'état de l'utilisateur pendant le sommeil analysé par l'unité d'analyse pour chaque maladie qui est étroitement liée au sommeil.

5. Système de surveillance du sommeil selon la revendication 3 ou la revendication 4, dans lequel
l'unité de surveillance inclut une pluralité d'unités de détection qui détectent chacune l'état de l'utilisateur pendant le sommeil, et
l'unité d'analyse analyse l'état de l'utilisateur pendant le sommeil en complétant mutuellement une pluralité d'informations de sommeil détectées par la pluralité d'unités de détection.

6. Système de surveillance du sommeil selon l'une quelconque des revendications 3 à 5, dans lequel
l'unité de surveillance acquiert des informations corporelles relatives au corps de l'utilisateur,
l'unité d'acquisition acquiert les informations corporelles auprès de l'unité de surveillance, et
l'unité d'analyse effectue une correction sur la base des informations corporelles et analyse l'état de l'utilisateur pendant le sommeil.
